# EUROPEAN PATENT SPECIFICATION

(11) **EP 2 665 468 B1**
(45) Date of publication and mention of the grant of the patent: **27.12.2017**
(21) Application number: 12703955.0
(22) Date of filing: 18.01.2012
(51) Int. Cl.: A61K 9/20, A61K 9/28, A61K 31/365

(54) **PHARMACEUTICAL COMPOSITIONS OF IMMUNOSUPPRESSANTS**
PHARMAZEUTISCHE ZUSAMMENSETZUNGEN VON IMMUNOSUPPRESSIVA
COMPOSITIONS PHARMACEUTIQUES D'IMMUNODÉPRESSEURS

(30) Priority: 18.01.2011 GB 201100786
(43) Date of publication of application: 27.11.2013
(73) Proprietor: EMS S.A., CEP-13186-901 Hortolandia, SP (BR)
(72) Inventor: SANTUS, Giancarlo, CEP-13186-901 Hortolandia, SP (BR); SOLDATI, Giuseppe, CEP-13186-901 Hortolandia, SP (BR)
(74) Representative: Bawden, Peter Charles
(86) International application number: PCT/EP2012/000200
(87) International publication number: WO 2012/097978

(56) References cited:
- WO-A1-2006/110802
- WO-A2-2006/024479
- CN-A- 1 863 509

## Description

### Field of the invention

This invention relates to pulsatile release of immunosuppressants, particularly mycophenolate salts, to provide good bioavailability and reduced gastrointestinal side effects.

### Background

Immunosuppressants are mainly used to prevent rejection in organ transplantation. They are known to have also other biological activities such as anti-inflammatory, antipsoriatic, antiviral and antitumoral activity. Immunosuppressants can function by blocking both B-and T-cells proliferation, by inhibiting inosine monophosphate dehydrogenase (IMPDH), a key enzyme involved in the purine synthesis. Immunosuppressants however have poor bioavailability.

Mycophenolic acid is a known immunosupressant but it has poor bioavailability. To overcome poor bioavailability of mycophenolic acid several attempts have been made, including the formation of ester prodrugs. US Patent 4,753,935 describes the most important of these esters: the morpholinoethyl ester, marketed as CELLCEPT® - ROCHE, which is absorbed and then cleaved to mycophenolic acid and achieves a remarkable improvement in bioavailability, but the technique has limitations due to side effects mainly at gastrointestinal level.

Another approach to overcome the gastrointestinal problems of mycophenolic acid that has been suggested is the development of salts of alkali metals, mainly sodium, in the form of enteric coated formulations. European Patent EP 892 640 describes, enteric coatings that remain intact for at least 2 hours in an acidic environment and then disintegrate at a pH of 6.8 that mimics intestinal juices. A limitation of this approach is in the variability linked to the different gastrointestinal pH of the patients. The most known of these preparation is marketed as Myfortic® EC tablets. This approach is described in PCT publications WO03/032978 and WO2007/093346.

A third approach is described in PCT publications WO2009/022355 and WO2006/024479, and involves extended release preparations of sodium mycophenolate where a gradual release of the active ingredient over time is obtained. In this case a limitation may be the bioavailability, as mycophenolic acid is known to have an optimal absorption window in the upper part of the intestine.

Pulsatile drug release is a known technique that delivers a burst of drug release within the body at one or more predetermined time intervals. Pulsatile release may be used to avoid drug degradation/irritation in the stomach or, for example, the ability to administer drugs released at different sites in the Gastro Intestinal tract etc. Pulsatile release can be achieved by employing tablets, capsules or pellets provided with a pH dependent or barrier membrane coating systems. Alternatively pulsatile release may be achieved by providing a water permeable membrane on a tablet, capsule, or pellet and providing a material within the tablet, capsule or pellet which expands or foams on contact with water. With this system once sufficient water has permeated the membrane the tablet, capsule or pellet will burst releasing the active drug ingredient.

US Patent 4,871,549 describes the "time-controlled explosion systems" in which drug release is caused by explosion of a membrane after a definite time period. This system is composed by various cores, layers, membranes and coating materials, which can be mixed in different ways in order to result in various drug release patterns. In this way sustained release preparations could be used to reduce frequency of dosing, to prevent undesirable side effects and to get optimum therapeutic efficiency.

US Patent 5,472,708 describes unit dosages forms for delivering drugs in a series of sequential and pulsatile release events, through a plurality of populations of coated pellets within a unit dosage form such as a capsule. By varying the proportion of the coating ingredients or coating thickness from one pellet population to another the release timing of the pellets can be controlled.

Proprietary Pulsatile Delivery Systems are also currently available from Watson Pharmaceuticals Inc ("DPHS" and "PPDS") and from Victory Pharma Inc ("PULSYS").

Although pulsatile release technologies are known, there is still a need for a less complex pulsatile release system which is compatible with the demands of immunosuppressants and particularly mycophenolate salts administration.

The present invention deals with a single pulse pulsatile drug delivery system obtained with a pH-independent, water insoluble but water permeable coating system applied on a swellable core containing an immunosupressant.

### Summary of the invention

The pharmaceutical composition of the present invention is a pulsatile formulation of an immunosuppressant with pH-independent polymer coatings for achieving an immediate release of immunosuppressants in the upper part of the intestinal tract, as defined by claim 1.

### Description of the invention

The present invention, as defined by claim 1, provides pulsatile compositions comprising the immunosupressant mycophenolate sodium as the active ingredient for once-a-day or twice-a-day administration wherein said composition provides a better patient treatment with good bioavailability and reduced gastrointestinal side effects.
In particular the present invention deals with single-pulse rupturable dosage forms. We have developed a dosage form with an inner compartment containing a swellable polymer and an external pH-independent, water insoluble but water permeable coating of said inner compartment. The inner compartment can be a tablet core, a pellet or a capsule.
Upon contact with gastro-intestinal fluids, water penetrates through the outer polymer coating, and/or the pores created in the film, and hydrates the swelling polymer of the inner compartment to cause it to swell.

This swelling of the polymer will develop a pressure inside the tablet, capsule or pellet which will rupture the external pH-independent coating leading to release of the drug. Before the coating rupture and release of the drug, there is a lag time controlled by the time required for the polymer of the inner compartment to swell sufficiently to cause rupture, by the permeability and mechanical properties of the external coating and by the dimensions of the pores inside the coating.
The terms "pH-independent polymer", "pH-independent coating" refer to the properties of certain polymers, or coating formulation, not to be affected by pH. This is different for example from "enteric coating" that, is pH dependent and that remains intact in artificial gastric fluid, such as HCl pH 1, but then disintegrates in artificial intestinal fluid, such as KH₂PO4 buffered solution pH 6.8.
The presence in the inner compartment of an appropriate swellable polymer is critical for the performance of the present pulsatile formulation. In case of a film coated tablet the inner compartment is the tablet core. Swellable polymers that are employed are derivatives of cellulose that have selective swelling characteristics and they are preferred as they swell more in basic environment than in acid one. These polymers are the so called cross-linked sodium carboxymethylcellulose (croscarmellose sodium) such as for example, Ac-Di-Sol®, Nymcel® etc. These polymers have been selected for their swelling characteristics.
When they are in contact with the gastric fluid the polymers start swelling and the swelling causes rupture of the pH-independent film with the release of the active ingredient.
One advantage of the preferred embodiments of this invention is that the pulsatile formulation allows good bioavailability and stability of an immunosuppressant such as mycophenolic acid without the need for enteric polymers.
Surprisingly in the preferred embodiment of the invention the innovative mycophenolic acid salt pulsatile formulation provides good stability and dissolution profile when compared with a reference product Myfortic.

The inner compartment is coated with pH-independent polymer coatings such as ethylcellulose e.g. ETHOCEL® from Dow, AQUACOAT® from FMC, or SURELEASE® from Colorcon. The coating can be applied in aqueous dispersion or in organic solvents. The coating may further include one or more components such as pore formers or plasticizers. The pore former is hydroxypropylmethylcellulose (HPMC) e.g. METHOCEL®. Suitable plasticizers are triethyl citrate, dibutylsebacate, triacetine etc. The plasticizer is present in amount 1 to 40% by weight and preferably 5-10% of the total weight of the coating.
The present invention may be used for the improved bioavailability of any immunosuppressant. Although it has been described in relation to the preferred immunosuppressant sodium mycophenolate it can be used with immunosuppressants such as cyclosporin, tacrolimus, voclosporin, monoclonal antibiotics and rapamycin or mixtures thereof..
The preferred sodium mycophenolate compositions of the invention are administered at a dose in the range of 0.5 to 2.0 g/day of sodium mycophenolate. A common dosage and administration is about 1.5 g/day divided twice daily. The recommended dose in adult renal transplantation is 720 mg twice daily (BID), preferably on an empty stomach, one hour before or two hours after food intake. In paediatric patients the dose must be adjusted according to body surface area, the dose being of 400 mg/m² up to a maximum of 720 mg BID.
The sodium mycophenolate compositions of the present invention can be administered in combination with other immunosuppressant such as for example conventional steroids, cyclosporine, tacrolimus, sirolimus, monoclonal antibodies, etc. The sodium mycophenolate compositions can be given in concomitant, sequential or separate administration with the other immunosuppressants.

The present invention will now be illustrated by the following examples. The formulation based on the system employed in the examples can be formed by any suitable method known in the art.

### Example 1. Preparation of pulsatile mycophenolate formulation (not according to the invention)

**Composition of the core**

| | |
|---|---|
| Sodium mycophenolate (corresponding to Mycophenolic acid 360 mg) | 384.8 mg |
| Lactose anhydrous | 110.5 mg |
| Croscarmellose sodium | 65.0 mg |
| Povidone K30 | 40.0 mg |
| Colloidal silicon dioxide | 13.2 mg |
| Magnesium stearate | 6.5 mg |
| Ethanol (removed during the process) | 50.0 mg |

**Composition of the film**

| | |
|---|---|
| Ethylcellulose | 9.75 mg |
| Hypromellose 5 cps | 4.5 mg |
| Triethylcitrate | 0.75 mg |
| Acetone/water (removed during the process) | 300.0 mg |

### Procedure

Mycophenolate sodium, colloidal silicon dioxide and povidone were sieved and blended for a few minutes.

The mixture of powders was kneaded with ethanol and the wet granulate was passed through a 2.0 mm sieve.

The granulate was dried in a static oven at 40 - 50°C and then passed through a 0.8 mm sieve.

Further excipients (lactose, croscarmellose sodium and magnesium stearate) were then added.

After blending, the final mixture was compressed to the target weight (17.0 x 7.2 mm capsule shaped concave punches).

The core tablets obtained were coated in a pan coater using a solution of ethylcellulose in acetone:water (90:10 w/w). Tablets bed temperature: 30-35°C.

### Example 2. Preparation of pulsatile mycophenolate formulation with aqueous film coating

**Core**

| | |
|---|---|
| Sodium mycophenolate (corresponding to Mycophenolic acid 360 mg) | 384.8 mg |
| Lactose anhydrous | 110.5 mg |
| Croscarmellose sodium | 80.0 mg |
| Povidone K30 | 25.0 mg |
| Colloidal silicon dioxide | 13.2 mg |
| Magnesium stearate | 6.5 mg |
| Ethanol (removed during the process) | 45.0 mg |

### Composition of the aqueous film

**Dry film (8 % total of the film)**

| | |
|---|---|
| Ethylcellulose dispersion Surelease® | 80% |
| Hypromellose (5 cps) | 15% |
| Triethylcitrate | 5% |

*Purified Water (92% total of the film)*

The core tablets of Example 2, were prepared with the same manufacturing method reported in the Example 1, and were then coated in a pan coater using an aqueous dispersion of ethylcellulose.

The film-coating suspension was maintained under gentle agitation during the process. Tablets bed temperature: 45°C.

Samples of tablets were collected at different times of spraying equivalent at 1 mg; 3 mg; 5mg; 10mg and 15mg of dry film and the dissolution was measured and it is reported in Example 4 point B.

### Example 3. Preparation of pulsatile formulation with coating without plasticizer triethylcitrate .

### Composition of the core

The core was prepared as in Example 2

### Composition of the film

**Dry film (8 % total of the film)**

| | |
|---|---|
| Ethylcellulose dispersion Surelease® | 85% |
| Hypromellose (5 cps) | 15% |

*Purified Water (92% total of the film)*

The core tablets of Example 3 were coated in a pan coater using the aqueous dispersion of ethylcellulose.

The film-coating suspension was maintained under gentle agitation during the process. Tablets bed temperature: 45°C.
Samples of tablets were collected at different times of spraying equivalent at 5mg and 10mg of dry film.

### Example 4. In vitro release

The dissolution test method applied was the FDA official method for mycophenolic acid delayed release tablets: *USP apparatus II* (paddle); 50 rpm; *Medium:* Acid stage: 0.1N HCl (120 min), Buffer stage: buffer solution pH 6.8 (60 min); *Volume* 750 ml (acid), 1000ml (buffer); *Sampling time* (min) 120 (acid) 10, 20, 30, 45 and 60 (buffer).
A) Dissolution test performed on film coated tablets of formulation 1 vs. the reference Myfortic EC.

| | Form. Example 1 (n=6) | Myfortic EC (n=12) |
|---|---|---|
| Sampling times (min) | % Released | % Released |
| 0 | 0 | 0 |
| 120 (acid stage) | 0 | 0 |
| 120 + 10 (buffer stage) | 4 | 1 |
| 120 + 20 ( " ) | 30 | 22 |
| 120 + 30 ( " ) | 56 | 58 |
| 120 + 45 ( " ) | 89 | 95 |
| 120 + 60 ( " ) | 98 | 102 |

The two release profiles are similar.
B) Dissolution test was performed on aqueous film coated tablets of example 2 at different amount of coating.
The results obtained are reported below:

| Sampling times (min) | % released at different amount of coating | | | | |
|---|---|---|---|---|---|
| | 1mg | 3mg | 5mg | 10mg | 15mg |
| 0 | 0 | 0 | 0 | 0 | 0 |
| 120 (acid stage) | 2 | 1 | 2 | 2 | 0 |
| 120 + 10 (buffer stage) | 29 | 23 | 14 | 10 | 5 |
| 120 + 20 ( " ) | 64 | 52 | 32 | 25 | 12 |
| 120 + 30 ( " ) | 88 | 73 | 52 | 49 | 18 |
| 120 + 45 ( " ) | 97 | 94 | 75 | 76 | 31 |
| 120 + 60 ( " ) | 101 | 102 | 89 | 90 | 44 |

As expected the release of mycophenolate decreases, as the amount of core coating increases.
C) Dissolution test was performed on aqueous film coated tablets of Example 3 at different amount of coating without plasticizer.

The results obtained are reported below:

| Sampling times (min) | % released at different amount of coating | |
|---|---|---|
| | 5mg | 10mg |
| 0 | 0 | 0 |
| 120 (acid stage) | 2 | 1 |
| 120 + 10 (buffer stage) | 21 | 12 |
| 120 + 20 ( " ) | 53 | 25 |
| 120 + 30 ( " ) | 82 | 38 |
| 120 + 45 ( " ) | 94 | 58 |
| 120 + 60 ( " ) | 100 | 77 |

As expected the release of mycophenolate decreases, increasing the amount of coating on the core.

### Example 5. Stability

The stability of the formulations prepared as described in Examples 1 to 3 was checked for assay, related substances and dissolution after 6 months at room temperature and in accelerated conditions, according to ICH guidelines, and there were no significant changes from initial results.

### Example 6. Biological activity (not according to the invention)

The biological activity of the compositions of the present invention vs. the reference commercial product Myfortic® can be assessed in standard clinical bioequivalence trials. For example, two tablets of 384.8 mg of sodium mycophenolate (equivalent to 360 mg mycophenolic acid) of Myfortic® and of the example 1 of the present invention may be administered to 24 stable renal transplant patients in a single dose, in an open label two-way cross over design, as reported by Arns W et al. in Clin Transplant 19, 199-206 (2005*).* Reduction in variability of AUC, Cₘₐₓ and Tₘₐₓ of Example 1 can be expected.

## Claims

1. A single pulse pulsatile release pharmaceutical composition wherein the active ingredient is an immunosuppressant, comprising a compartment containing 62,1 wt% of a mycophenolate sodium salt as immunosuppressant, 12,9 wt% of croscarmellose sodium as water soluble polymer, 17.8 wt% of lactose, 4.0 wt% of povidone, 2,1 wt% of colloidal silicon dioxide, and 1,0 wt% of magnesium stearate, all based on the total weight of the compartment, said compartment being coated with either
(i) 0.16 wt% or 0.48 wt% of a dry film, based on the dry weight of the film relative to the total weight of the immunosuppressant comprising compartment, with this film consisting of 80 wt% of ethylcellulose as pH-independent water insoluble but water permeable polymer and, 15 wt% of hydroxypropylmethyl cellulose as the pore forming substance , and 5 wt% of triethylcitrate as plasticizer; or with
(ii) 0.81 wt% of a dry film, based on the dry weight of the film relative to the total weight of the immunosuppressant comprising compartment, with this film consisting of 85 wt% of ethylcellulose as pH-independent water insoluble but water permeable polymer, and 15 wt% of hydroxypropylmethyl cellulose as pore forming substance
wherein upon contact of the water swellable polymer with gastric fluid it swells to cause rupture of the pH-independent film with single pulse release of the mycophenolate salt.

## Patentansprüche

1. Pharmazeutische Zusammensetzung zur pulsierenden Einzelimpulsfreisetzung, wobei die aktive Ingredienz ein Immunsuppressivum ist, das ein Kompartiment umfasst, das umfasst: 62,1 Gewichtsprozent eines Mycophenolat-Natriumsalzes als Immunsuppressivum, 12,9 Gewichtsprozent Croscarmellose-Natrium als wasserlösliches Polymer, 17,8 Gewichtsprozent Laktose, 4,0 Gewichtsprozent Povidon, 2,1 Gewichtsprozent kolloidales Siliziumdioxid und 1,0 Gewichtsprozent Magnesiumstearat, jeweils basierend auf dem Gesamtgewicht des Kompartiments, wobei das Kompartiment ummantelt ist, entweder mit
(i) 0,16 Gewichtsprozent oder 0,48 Gewichtsprozent eines Trockenfilms, basierend auf dem Trockengewicht des Films relativ zu dem Gesamtgewicht des Kompartiments, das das Immunsuppressivum umfasst, wobei der Film aus 80 Gewichtsprozent Ethylzellulose als pH-unabhängiges wasserunlösliches, aber wasserdurchlässiges, Polymer und 15 Gewichtsprozent Hydroxypropylmethylzellulose als die porenbildende Substanz und 5 Gewichtsprozent Triethylzitrat als Weichmacher besteht; oder mit
(ii) 0,81 Gewichtsprozent eines Trockenfilms, basierend auf dem Trockengewicht des Films relativ zu dem Gesamtgewicht des Kompartiments, dass das Immunsuppressivum umfasst, wobei dieser Film aus 85 Gewichtsprozent Ethylzellulose als ein pH-unabhängiges wasserunlösliches, aber wasserdurchlässiges, Polymer und 15 Gewichtsprozent Hydroxypropylmethylzellulose als eine porenbildende Substanz besteht,
wobei es, nach einem Kontakt des wasserschwellbaren Polymers mit Magenflüssigkeit anschwillt, um eine Ruptur des pH-unabhängigen Films mit einer Einzelimpulsfreisetzung des Mycophenolat-Salzes zu verursachen.

## Revendications

1. Composition pharmaceutique à libération pulsatile à impulsion unique dans laquelle le principe actif est un immunosuppresseur, comprenant un compartiment contenant 62,1 % en poids d'un sel de sodium de mycophénolate en tant qu'immunosuppresseur, 12,9 % en poids de croscarmellose sodique en tant que polymère hydrosoluble, 17,8 % en poids de lactose, 4,0 % en poids de povidone, 2,1 % en poids de dioxyde de silicium colloïdal, et 1,0 % en poids de stéarate de magnésium, tous sur la base du poids total du compartiment, ledit compartiment étant enrobé avec :
(i) 0,16 % en poids ou 0,48 % en poids d'un film sec, sur la base du poids sec du film par rapport au poids total du compartiment comprenant l'immunosuppresseur, ce film étant constitué par 80 % en poids d'éthylcellulose en tant que polymère insoluble dans l'eau mais perméable à l'eau indépendamment du pH, 15 % en poids d'hydroxypropylméthylcellulose en tant que substance de formation de pore, et 5 % en poids de citrate de triéthyle en tant que plastifiant ; ou avec
(ii) 0,81 % en poids d'un film sec, sur la base du poids sec du film par rapport au poids total du compartiment comprenant l'immunosuppresseur, ce film étant constitué par 85 % en poids d'éthylcellulose en tant que polymère insoluble dans l'eau mais perméable à l'eau indépendamment du pH, et 15 % en poids d'hydroxy-propylméthylcellulose en tant que substance de formation de pore,
dans laquelle, lors du contact du polymère gonflant dans l'eau avec le liquide gastrique, il gonfle pour provoquer la rupture du film indépendant du pH avec une libération à impulsion unique du sel de mycophénolate.
